# EUROPÄISCHE PATENTSCHRIFT

(11) **EP 2 138 202 B1**
(45) Veröffentlichungstag und Bekanntmachung des Hinweises auf die Patenterteilung: **24.03.2010**
(21) Anmeldenummer: 08011322.8
(22) Anmeldetag: 23.06.2008
(51) Int. Cl.: A61M 39/10, F16L 37/12

(54) **Anschlussteil zur Verbindung mit einem genormten Luer-Lock-Anschluss**
Connecting part for connecting with a standardised luer lock connection
Elément de raccordement destiné à être raccordé à un raccord Luer Lock normalisé

(43) Veröffentlichungstag der Anmeldung: 30.12.2009
(73) Patentinhaber: Logica, 23758 Oldenburg (DE)
(72) Erfinder: Forberg, Hans-Jürgen, 23758 Oldenburg (DE)
(74) Vertreter: Vollmann, Heiko

(56) Entgegenhaltungen:
- EP-A- 0 953 365
- US-A- 4 588 402
- US-A- 4 668 217
- US-A1- 2007 088 325
- US-B2- 6 585 229

## Beschreibung

Die Erfindung betrifft ein Anschlussteil zur Verbindung mit einem genormten Luer- oder Luer-Lock-Anschluss mit den im Oberbegriff des Anspruchs 1 angegebenen Merkmalen.

In der Medizintechnik werden zur fluiddichten Anbindung medizinischer Geräte und Instrumente, z.B. von Infusionspumpen, häufig so genannte Luer-Verbindungen genutzt. Dabei handelt es sich um Kegelverbindungen, deren konische Flächen eine 6-prozentige Steigung aufweisen. Typischerweise werden die Anschlussteile dieser Verbindungen aus Kunststoff durch Spritzgießen gefertigt. Ein Anschlussteil gemäß des Oberbegriffs des Anspruchs 1 ist bekannt, siehe US 2007 088325 A1.

Grundsätzlich haben sich diese Verbindungen in der Medizintechnik bewährt, es kann jedoch unter ungünstigen Umständen zu Undichtigkeiten kommen. Zum einen können Maß- oder Formabweichungen bezüglich der Luer-Kegel eine dichtende Anlage der Kegelmantelflächen erschweren. Eine weitere Ursache für Undichtigkeiten kann in rauen oder beschädigten, beispielsweise verkratzen, Mantelflächen der Luer-Kegel liegen. Ferner kann auch das viskoelastische Fließen des Kunststoffs zu einer Lockerung der Luer-Verbindung und damit zu Undichtigkeiten führen, was insbesondere bei längeren Lagerzeiten oder bei der Lieferung von Geräten mit vorkonnektierten Luer-Anschlüssen auftritt.

In der Praxis wird häufig versucht, die Dichtigkeit einer Luer-Verbindung zu erhöhen, indem die Anschlussteile mit erhöhter Fügekraft zusammengefügt werden. Innerhalb der Verbindungsstücke, insbesondere in demjenigen Anschluss mit Luer-Innenkegel, treten dabei deutlich erhöhte Zugspannungen auf. Durch die daraus resultierenden Risse oder Brüche wird die Verbindung oftmals unbrauchbar.

Es ist daher Aufgabe der Erfindung, ein Anschlussteil mit einer verbesserten fluiddichten Anbindung zu schaffen.

Diese Aufgabe wird durch ein Anschlussteil mit den im Anspruch 1 angegebenen Merkmalen gelöst. Bevorzugte Ausführungsformen ergeben sich aus den Unteransprüchen, der nachfolgenden Beschreibung und der Zeichnung.

Das erfindungsgemäße Anschlussteil ist ein Anschlussteil zur Verbindung mit einem genormten Luer- oder Luer-Lock-Anschluss und weist einen konischen Bereich auf, der zur Anlage an der konischen Anschlussfläche des genormten Luer- oder Luer-Lock-Anschlusses ausgebildet ist. Dabei ist an dem konischen Bereich eine Dichtung angeordnet. In einer Verbindung mit dem erfindungsgemäßen Anschlussteil wirkt diese Dichtung daher zusätzlich zur dichtenden Anlage des konischen Bereichs an der konischen Gegenanlagefläche. Mit dieser Dichtung können Maß- und Formabweichungen sowie Beschädigungen der Anlageflächen eines angeschlossenen Luer- oder Luer-Lock-Anschlusses ausgeglichen werden, indem sich die Dichtung entsprechend verformt.

Die Dichtung, die elastisch ausgestaltet ist, weist ein gutes Rückstellverhalten auf. Bei der Verbindung des Anschlussteils mit einem Luer- bzw. Luer-Lock-Anschluss (im Folgenden als Gegenanschlussteil bezeichnet) verhindert die Dichtung darüber hinaus eine versehentliche Lockerung der Verbindung. Ferner ist schon bei geringer Fügekraft eine dichte Verbindung des erfindungsgemäßen Anschlussteils mit einem Gegenanschlussteil gegeben. Auch ist daher ein geringerer Kraftaufwand zum Lösen der Verbindung nötig, es verringert sich damit deutlich die Gefahr, das Anschlussteil bzw. das Gegenanschlussteil durch einen zu hohen Kraftaufwand und daraus herrührenden Rissen bzw. Bruchstellen zu beschädigen. Weiterhin zeigen Anschlussteil und Gegenanschlussteil bei einer geringeren Fügekraft eine deutlich verringerte Neigung, sich bei der Verbindung bzw. beim Lösen der Verbindung zu verkeilen. Ein gutes Rückstellverhalten der elastischen Dichtung vermeidet zudem das viskoelastische Fließen der Kunststoffe. Daher ist ein Nachkonnektieren der Verbindungsteile, etwa durch nachträgliches Drehen bzw. Drücken der Verbindungsstücke, nicht erforderlich. Vorzugsweise ist der konische Bereich als Mantelfläche eines Kegelstumpfes ausgebildet, die zur Anlage an einen Luer-Kegel ausgebildet ist. Diese Mantelfläche ist dabei nicht notwendigerweise als eine einzige zusammenhängende Flöche ausgebildet, sondern kann auch aus mehreren Flächenabschnitten bestehen, die beispielsweise durch eine als Dichtring ausgestaltete Dichtung voneinander getrennt oder segmentiert sind.

Bei dem erfindungsgemäßen Anschlussteil handelt es sich um ein männliches Anschlussteil, weiches zur Verbindung mit einem genormten weiblichen Luer-Anschluss ausgebildet ist.

Die Dichtung ist als vollumfänglich am konischen Bereich angeordneter Dichtring ausgebildet. Insbesondere ist dieser Dichtring koaxial zur Längsachse des konischen Bereichs orientiert. Auf diese Weise lässt sich entlang des gesamten Umfangs der zur Anlage kommenden konischen Bereiche von Anschlussteil und Gegenanschlussteil eine hohe Dichtwirkung erzielen. Ein rotationssymmetrischer Aufbau des Anschlussteils im Anschlussbereich ermöglicht zudem die einfache Verbindbarkeit des Anschlussteils mit dem Gegenanschlussteil.

Die Dichtung ist am freien axialen Ende des konischen Bereichs angeordnet. Dieses freie axiale Ende des Anschlussteils kommt mit einer Anlagefläche des Gegenanschlussteils zur Anlage, sobald sich die konischen Bereiche von Anschlussteil und Gegenanschlussteil ausreichend überlappen. Durch diese Ausgestaltung kann somit eine zuverlässige Dichtung leicht gewährleistet werden.

Vorzugsweise ist die Dichtung aus einem Elastomer, vorzugsweise einem Silikonkautschuk oder einem thermoplastischen Elastomer, gebildet. Eine solche elastische Dichtung lässt sich kostengünstig fertigen und weist darüber den Vorteil auf, dass diese ein gutes Rückstellverhalten hat. Wird also eine Verbindung mit dem erfindungsgemäßen Anschlussteil gelöst, so geht die Dichtungskontur nahezu in ihre ursprüngliche Form zurück. Auf diese Weise kann auch bei einem wiederholten Wechsel des Gegenanschlussteils eine zuverlässig dichtende Verbindung mit dem Anschlussteil erfolgen.

Bevorzugt ist bei dem erfindungsgemößen Anschlussteil die Dichtung stoffschlüssig am konischen Bereich angeformt. Auf diese Weise ist die Dichtung mit dem übrigen Anschlussteil einstückig bzw. einteilig ausgestaltet. Ein Verrutschen bzw. ein Verlieren der Dichtung wird damit wirksam vermieden. Entsprechend ist es beim Einsatz des erfindungsgemäßen Anschlussteils nicht erforderlich, die Dichtung geeignet zu positionieren oder vor einer Verbindung des Anschlussteils mit einem weiteren Anschlussteil die Dichtung an das Anschlussteil anzubringen.

In einer bevorzugten Ausgestaltung ist das Anschlussteil zusammen mit der Dichtung als Zweikomponenten-Spritzgussteil ausgebildet. Auf diese Weise kann zum einen unaufwändig eine stoffschlüssige Verbindung der Dichtung mit dem konischen Bereich des Anschlussteils realisiert werden. Zum anderen sind solche Anschlussteile im Kunststoffspritzgießverfahren kostengünstig herstellbar.

Ferner bevorzugt ist das Anschlussteil Teil eines Bauteils mit einem Rückschlagventil. Insbesondere bei Infusionsanwendungen sind Komponenten häufig mit Rückschlagventilen versehen. Eine Integration eines solchen Rückschlagventils in ein erfindungsgemäßes Anschlussstück bietet hier einen erheblichen Vorteil. Liegen Rückschlagventil und Anschlussstück als integrales Bauteil vor, so lassen sich diese Komponenten kostengünstiger produzieren, einfacher montieren sowie vereinfacht handhaben.

Weiter bevorzugt ist das Anschlussteil Teil eines Bauteils, welches als Spritzgussteil ausgebildet ist. In der Medizin-, insbesondere der Infusionstechnik, sind überdies eine Vielzahl von Komponenten durch Spritzgießen gefertigt. Darüber hinaus weisen diese Komponenten häufig Luer- bzw. Luer-Lock-Anschlüsse auf. Werden diese gemeinsam mit dem erfindungsgemößen Anschlussteil als einteiliges bzw. einstückiges Bauteil durch Spritzgießen gefertigt, lassen sich Produktionsschritte und damit auch Fertigungskosten einsparen. Ferner ist auch die Handhabung, die Montage bzw. die Wartung solcher einteiligen bzw. einstückigen Bauteile erheblich vereinfacht.

Nachfolgend ist die Erfindung anhand von in der Zeichnung dargestellten Ausführungsbeispielen beschrieben. Es zeigen:
- Fig. 1: ein Anschlussteil, welches als weibli- ches Anschlussteil zur Verbindung mit einem männlichen Luer-Anschluss ausgebildet ist, im Längsschnitt,
- Fig. 2: das Anschlussteil gemäß Fig. 1 in einer Verbindung mit ei- nem männliche Luer-Anschluss im Längsschnitt,
- Fig. 3: ein weiteres Anschlussteil, welches als weibliches Anschlussteil zur Verbindung mit einem männli- chen Luer-Lock-Anschluss ausgebildet ist, im Längsschnitt,
- Fig. 4: das Anschlussteil gemäß Fig. 3 in einer Verbindung mit ei- nem männlichen Luer-Lock-Anschluss im Längsschnitt,
- Fig. 5: ein erfindungsgemäßes Anschlussteil, welches als männliches Anschlussteil zur Verbindung mit einem weibli- chen Luer-Lock-Anschluss ausgebildet ist, im Längsschnitt und
- Fig. 6: das Anschlussteil gemäß Fig. 5 in einer Verbindung mit ei- nem weiblichen Luer-Lock-Anschluss im Längsschnitt.

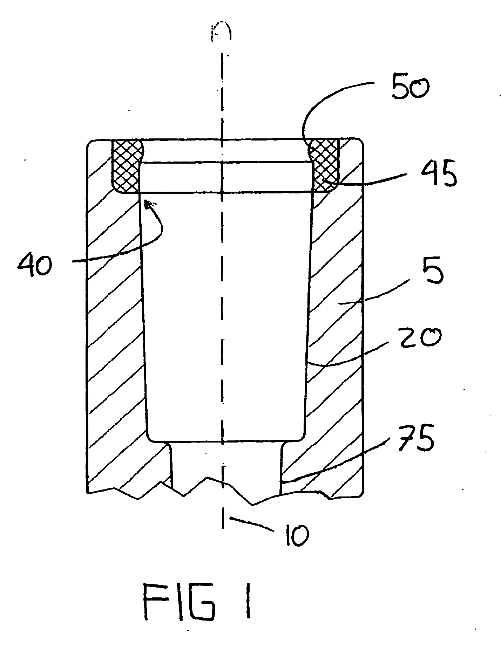

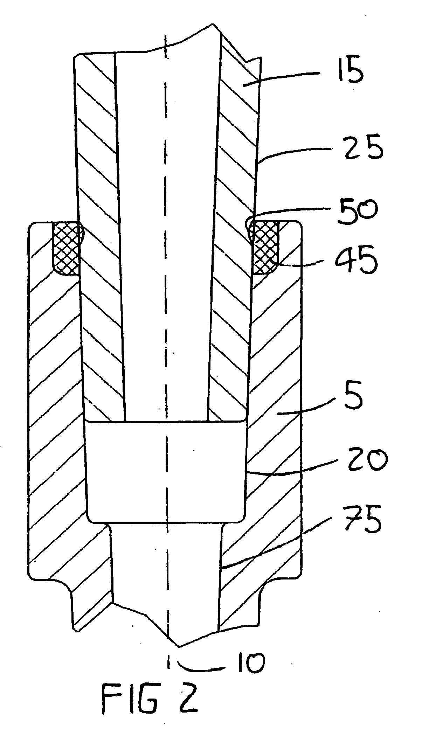

Ein in den Fig. 1 und 2 dargestelltes Anschlussteil 5 ist als weibliches Anschlussteil ausgestaltet. Dabei weist das Anschlussteil 5 im Wesentlichen Zylindersymmetrie bezüglich seiner Längsachse 10 auf. Das Anschlusssteil 5 ist dabei zur Verbindung mit einem genormten Luer-Anschluss 15 als Gegenonschiussteil ausgestaltet. Dazu weist das Anschlussteil 5 einen konischen Bereich 20 auf, welcher in dem gezeigten Ausführungsbeispiel eine Anschlussöffnung 20 zur Verbindung mit der konischen Anschlussfläche 25 eines genormten Luer-Anschlusses 15 bildet. Der konische Bereich 20 ist als Innenkegel 20 ausgebildet, d. h. als kegelstumpfartige Ausnehmung mit sich weitender Öffnung in Richtung des freien axialen Endes 40 des Anschlussteils 5. Die konischen Flächen 20 des konischen Bereichs 20 besitzen eine 6-prozentige Steigung, sodass der konische Bereich 20 mit der konischen Anschlussfläche 25 des Luer-Anschlusses 15 flächig zur Anlage kommen kann.

An der konischen Anschlussfläche 20 des Anschlussteils 5 ist am freien axialen Ende 40 des Anschlussteils 5 eine Dichtung 45 stoffschlüssig angebunden. Die Dichtung 45 ist als koaxial zum Innenkegel orientierter Dichtring 45 ausgestaltet. Die Dichtung 45 besteht aus thermoplastischem Elastomer, während das übrige Anschlussteil 5 aus bei Raumtemperatur starrem thermoplastischem Kunststoff gefertigt ist. Dabei sind Dichtung 45 und Anschlussteil 5 zusammen im Zweikomponenten-Spritzgießverfahren gefertigt. Das Dichtungselement 45 weist zu einer geeigneten Anpassung an den Luer-Außenkegel 25 eine als Dichtwulst 50 ausgestaltete nach innen vorspringende Dichtungskontur auf, welche bei gelöster Verbindung geringfügig in den konischen Öffnungsbereich 20 des Anschlussstücks 5 hineinragt. Auf diese Weise hat das Anschlussstück 5 stets dichtenden Kontakt mit dem Außenkegel 25 des Luer-Anschlusses 15.

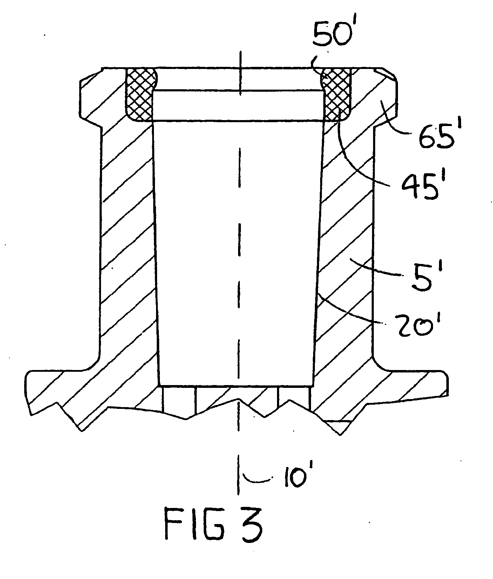

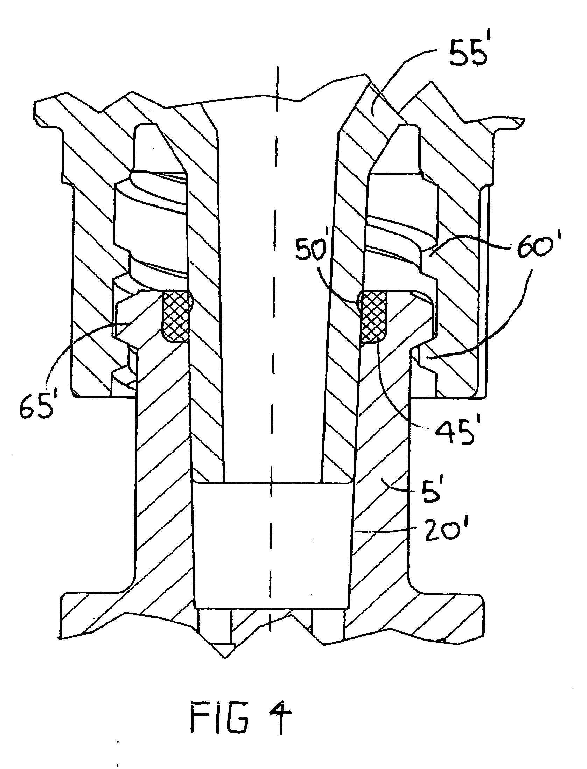

Das in den Fig. 3 und 4 dargestellte Anschlussteil 5' ist ebenfalls ein weibliches Anschlussteil 5' und zur Verbindung mit einem Luer-Lock-Anschluss 55' ausgestaltet. Dabei weist das Anschlussteil 5' soweit es den Innenteil angeht dieselben Merkmale wie das in den Fig. 1 und 2 dargestellte Anschlussteil 5 auf. Darüber hinaus besitzt dieses Anschlussteil jedoch zusätzliche Merkmale für eine verriegelbare Verbindung mit dem Luer-Lock-Anschluss.

Zur Verriegelung weist der Luer-Lock-Anschluss 55' bekanntermaßen ein Innengewinde 60' auf, welches koaxial zu dem Luer-Kegel 25' angeordnet ist und diesen vollumfänglich umgibt. Zur Verriegelung mit diesem Innengewinde 60' des Luer-Lock-Anschlusses 55' ist das Anschlussteil 5' mit 2 radiale Zungen zum Gewindeeingriff 65' versehen, welche zum Eingriff mit dem Gewinde 60' des Luer-Lock-Anschlusses 55' bestimmt sind. Diese Zungen 65' sind gleich jenen ausgestaltet, welche bei einem genormten Luer-Anschluss zur Verbindung mit einem Luer-Lock-Anschluss 55' dienen. So sind die Zungen 65' im Wesentlichen diametral angeordnet. Dabei befinden sich die Zungen 65' am freien axialen Ende 40' des Anschlussteils 5', also an jenem Ende, an welchem die Dichtung 45' befestigt ist. Die Zungen 65' kragen dabei in radialer Richtung nach außen aus. Somit kann das Anschlussteil 5' in den Luer-Lock-Anschluss 55' eingeschraubt werden, bis eine hinreichend dichte Anlage des konischen Bereiches 20' bzw. der Dichtung 45' mit dem Luer-Außenkegel 25' des Luer-Lock-Anschlusses 55' erreicht ist.

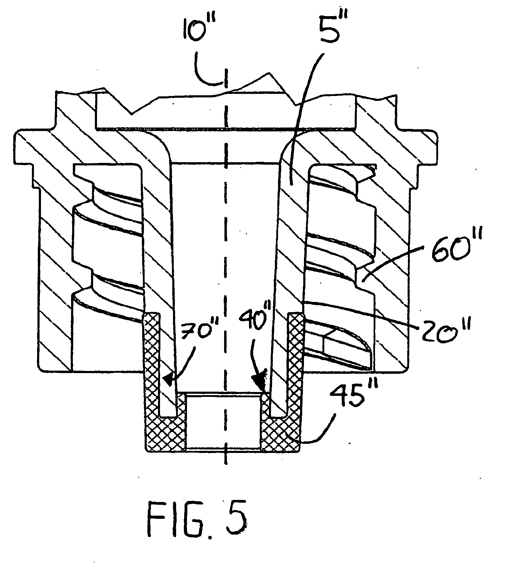

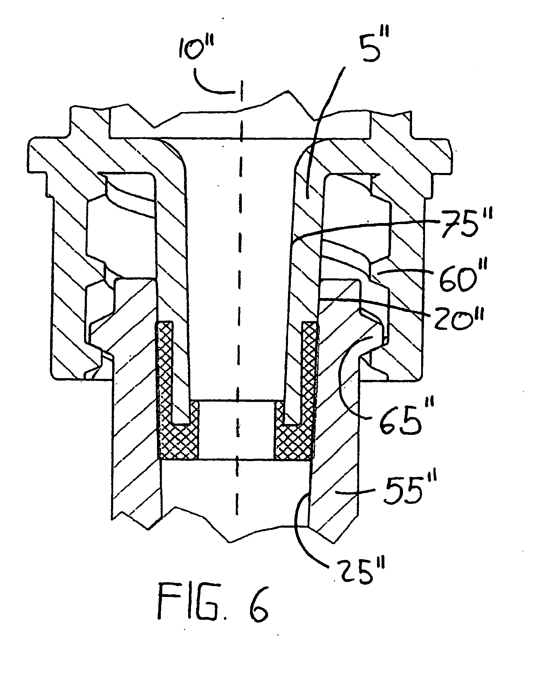

Ein erfindungsgemäßes Anschlussteil 5" ist in den Fig. 5 und 6 dargestellt. Dieses ist als männliches Anschlussteil 5" und zur Verbindung mit einem weiblichen Luer-Lock-Anschluss 55" ausgebildet. Das Anschlussteil 5" weist zum Anschluss an den Luer-Lock-Anschluss 55" einen konischen Bereich 20" auf. Der konische Bereich 20" ist als Außenkegel 20" ausgebildet, d. h. er besitzt ein kegelstumpfförmiges Profil mit einem sich in Richtung des freien axialen Endes 40" des Anschlussteils 5" verjüngenden Durchmesser. Dieser Außenkegel ist damit passend zu einem Innenkegel eines genormten weiblichen Luer-Lock-Anschlusses 55" ausgestaltet, an welchem er flächig zur Anlage kommen kann. Am freien axialen Ende 40" des Anschlussteils 5" ist eine umfänglich angeordnete Ringdichtung 45" ausgebildet. Daher ist bereits ein geringer axialer Überlapp von Anschlussteil 5" mit einem weiblichen Luer-Lock-Anschlussteil 55" zur dichtenden Anlage hinreichend. Die Ringdichtung 45" ist dergestalt am axialen Ende 40" des Anschlussteils 5" aufgebracht, dass am axialen Ende 40" die Mantelfläche des Außenkegels 20" in die Außenfläche der Dichtung 45" übergeht. Dazu weist der Außenkegel 20" am axialen Ende 40" eine umfängliche Ausnehmung 70" auf, welche sich bis zum axialen Ende 40" erstreckt und in welche das Material der Dichtung 45" aufgebracht ist. Die Dichtung 45" erstreckt sich über die Querschnittsfläche am axialen Ende 40" des konusförmigen Bereichs 20" hinweg und ragt geringfügig in den Endbereich des Innenschaftes 75" des Anschlussteils 5" hinein.

Zur Verriegelung mit einem weiblichen Luer-Lock-Anschluss 55" weist das Anschlussstück 5" ein den konischen Bereich 20" umfänglich umgebendes und koaxial orientiertes Innengewinde 60" auf, mit welchem korrespondierende Zungen 65" des weiblichen Luer-Lock-Anschlusses 55" in Eingriff treten können (siehe Fig. 6). Auch dieses Anschlussteil 5" ist als einteiliges bzw. einstückiges Bauteil gemeinsam mit der Dichtung 45" als Zweikomponenten-Spritzgussteil gefertigt.

### Bezugszeichenliste

- 5. 5', 5" -: Anschlussteil
- 10, 10', 10" -: Längsachse des Anschlussteils
- 15 -: Anschlussteil des genormten Luer- Anschlusses
- 20, 20', 20" -: konischer Bereich des Anschlussteils
- 25, 25' -: konische Anschlussfläche des genormten Luer-Anschlusses
- 25" -: Konische Anschlussfläche eines genormten Luer-Lock-Anschlusses
- 40,40', 40" -: freies axiales Ende des Anschlussteils
- 45, 45', 45", -: Dichtung
- 50, 50' -: Dichtwulst
- 55', 55" -: Anschlussteil eines genormten Luer-Lock- Anschlusses
- 60', 60" -: Innengewinde
- 65', 65" -: Zungen zum Gewindeeingriff
- 70" -: Ausnehmung
- 75', 75" -: Innenschaft des Anschlussteils

## Patentansprüche

1. Anschlussteil (5") zur Verbindung mit einem genormten Luer- oder Luer-Lock-Anschluss (55"), welches einen konischen Bereich (20") aufweiset, der zur Anlage an der konischen Anschlussfläche (25") des genormten Luer- oder Luer-Lock-Anschlusses (55") ausgebildet ist, **dadurch gekennzeichnet, dass** es als männliches Anschlussteil (5") ausgebildet ist, dass an dem konischen Bereich (20") eine Dichtung (45") angeordnet ist und dass die Dichtung (45") als vollumfänglich am konischen Bereich (20") angeordneter Dichtring (45") ausgebildet und am freien axialen Ende (40") des konischen Bereichs (20") angeordnet ist.

2. Anschlussteil (5") nach Anspruch 1, **dadurch gekennzeichnet, dass** die Dichtung (45") aus einem Elastomer, insbesondere Silikonkautschuk, oder einem thermoplastischen Elastomer gebildet ist.

3. Anschlussteil (5") nach Anspruch 1 oder 2, **dadurch gekennzeichnet, dass** die Dichtung (45") stoffschlüssig am konischen Bereich (20") angeordnet ist.

4. Anschlussteil (5") nach einem der vorangehenden Ansprüche, **dadurch gekennzeichnet, dass** es zusammen mit der Dichtung (45") als Zweikomponenten-Spritzguss-Teil ausgebildet ist.

5. Anschlussteil (5") noch einem der vorangehenden Ansprüche, **dadurch gekennzeichnet, dass** es Teil eines Bauteils mit einem Rückschlagventil ist.

6. Anschlussteil (5") nach einem der vorangehenden Ansprüche, **dadurch gekennzeichnet, dass** es Teil eines als Spritzgussteil ausgebildeten Bauteils ist.

## Claims

1. A connection part (5") for connection to a standardised luer connection or luer lock connection (55"), which comprises a conical region (20"), which is designed for bearing on the conical connection surface (25") of the standardised luer connection or luer lock connection (55"), **characterised in that** it is designed as a male connection part (5"), that a seal (45") is arranged on the conical region (20") and that the seal (45") is designed as a sealing ring (45") arranged over the whole periphery on the conical region (20") and is arranged at the free axial end (40") of the conical region (20").

2. A connection part (5") according to claim 1, **characterised in that** the seal (45") is formed of an elastomer, in particular silicon rubber or of a thermoplastic elastomer.

3. A connection part (5") according to claim 1 or 2, **characterised in that** the seal (45") is arranged with a material fit on the conical region (20").

4. A connection part (5") according to one of the preceding claims, **characterised in that** together with the seal (45"), it is designed as a two-component injection moulded part.

5. A connection part (5") according to one of the preceding claims, **characterised in that** it is part of a component with a return valve.

6. A connection part (5") according to one of the preceding claims, **characterised in that** it is part of a component designed as an injection moulded part.

## Revendications

1. Connecteur (5") pour le raccordement à un raccord Luer ou Luer Lock normalisé (55") qui comporte une zone conique (20") conçue de façon à venir s'appuyer contre la surface de raccordement conique (25") du raccord Luer ou Luer Lock normalisé (55"), **caractérisé en ce qu'**il est conçu sous forme d'un connecteur (5") de type mâle, **en ce qu'**un joint (45") est disposé au niveau de la zone conique (20") et **en ce que** le joint (45") est conçu sous forme d'une bague d'étanchéité (45") disposée en faisant complètement le tour de la zone conique (20") et **en ce qu'**il est placé au niveau de l'extrémité axiale libre (40") de la zone conique (20").

2. Connecteur (5") selon la revendication 1, **caractérisé en ce que** le joint (45") est en élastomère, en particulier en caoutchouc siliconé, ou en élastomère thermoplastique.

3. Connecteur (5") selon la revendication 1 ou 2, **caractérisé en ce que** le joint (45") est disposé contre la zone conique (20") de façon étanche aux matières.

4. Connecteur (5") selon l'une des revendications précédentes, **caractérisé en ce qu'**il est conçu conjointement avec le joint (45") en une pièce moulée par injection à deux composants.

5. Connecteur (5") selon l'une des revendications précédentes, **caractérisé en ce qu'**il fait partie d'un élément de construction comportant un clapet antiretour.

6. Connecteur (5") selon l'une des revendications précédentes, **caractérisé en ce qu'**il fait partie d'un élément de construction conçu sous forme d'une pièce moulée par injection.
